# EUROPEAN PATENT APPLICATION

(11) **EP 1 808 790 A1**
(43) Date of publication of application: **18.07.2007**
(21) Application number: 05292703.5
(22) Date of filing: 15.12.2005
(51) Int. Cl.: G06F 19/00

(54) **Method of determining risk scores**

(71) Applicant: Statlife, 75007 Paris (FR)
(72) Inventor: Ragusa, Stéphane, 75007 Paris (FR)
(74) Representative: Hirsch & Associés

(57) **Abstract**

A method of determining pathological condition risk scores for a given individual belonging to a final population comprises the steps of:
- selecting a prospective cohort from an initial population;
- estimating an individual relative module of risk scores for the individual from the prospective cohort, said module comprising at least one risk score associated with at least one pathological condition variable;
- multiplying each risk score of the individual relative module by the incidence of each pathological condition in the final population of the individual.

This method allows a more reliable estimation of the pathological condition risk scores for individuals belonging to populations different from the initial population of the prospective cohort.

## Description

### CONTEXT OF THE INVENTION

The present invention relates to a method of determining pathological condition risk scores for a given individual based on a prospective cohort. Such a method does not provide a medical diagnosis, but constitutes a tool of assistance to the physician for estimating the risks of future pathological conditions of an individual who is still healthy, and allows the physician to adapt the preventive treatments possibly necessary.

### STATE OF THE ART

Assessment of the risks associated with an individual is a very common approach in financial sectors such as insurance or credit establishment. This approach of assessment of the risks associated with an individual is also of interest to the medical sector, which sees in this risk assessment a means of adapting preventive measures to each individual as a function of his or her risk scores. For example, an individual exhibiting a high risk score for a certain cancer could have more expensive diagnostic methods applied to him or her; and an individual exhibiting a high risk score for cardiovascular stroke could be prescribed blood lipid-lowering treatments even before the appearance of first symptoms and without a particularly high level of each of the risk factors.

Such methods of assessing medical risks are difficult to perform. This is because, while in the insurance or credit establishment sectors the statistical methods for assessing the risks of a new individual are quite simple given the abundance of data, the same is not true in the medical sector. Medical databases are rare and expensive, and are often small in size, requiring more complex methods for limiting to a maximum the loss of information in the course of the statistical treatment.

Today, several types of data are theoretically available for assessing pathological condition risk scores for an individual still healthy. These medical data can be found in the files of insurance companies; these data are abundant but may be biased due to a lack of objectivity of the individuals insured. Other medical data are available in hospital files; these data concern the sick population and are therefore partial. Data also exist that are provided by epidemiological inquiries referred to as "a case-control study", where individuals who are sick and not sick are questioned retrospectively regarding their prior lifestyle; one is often confronted with a selective memory which tends to minimize certain risk factors such as smoking. Epidemiological inquiries referred to as "prospective" also exist, where a "prospective cohort" is constructed, i.e. an entire population is considered, for which individual parameters (lifestyle, diet, etc.) are recorded and which is followed for many years in order to list the pathological conditions developed.

Prospective cohorts are today the most adapted tools for estimating pathological condition risk scores. For a new individual, an investigation of his or her closest neighbours within the prospective cohort makes it possible to estimate said individual's risk of developing such or such a pathological condition according to the number of individuals of the cohort exhibiting substantially the same individual parameters and having developed said pathological condition.

Such a method of estimating risk scores based on a prospective cohort requires having such a cohort and especially requires this cohort to contain a sufficient number of individuals to reveal sufficient sick individuals. In fact, if a prospective cohort of 10 000 individuals is considered, the number of individuals having developed a pathological condition after 10 years may be of the order of a few hundred for cardiovascular diseases or breast, lung or colon cancer, and of the order of a few tens for the other less prevalent pathological conditions. Prospective cohorts of 100 000 individuals are therefore more suitable, but only a few examples of such cohorts exist throughout the world, and some have few recorded parameters.

Physicians today mainly have models for estimating risk scores for two major pathological conditions: cardiovascular risks with the scores derived from the Framingham cohort and the risk of breast cancer with the Gail model derived from a case-control study.

The Gail model is presented in the publication by M.H. Gail et al., "Projecting individualized probabilities of developing breast cancer for white females who are being examined annually", J. Nat. Cancer Inst. 1989 Dec. 20; 81(24): 1879-86; this model is used to assess a risk before a possible mastectomy or as a criterion for identifying patients before their inclusion in a clinical trial and as an aid to choosing a therapeutic prescription. The applications of the Gail model are described, for example, in the publications by Freedman A.N. et al., "Estimates of the number of US women who could benefit from taxomifen for breast cancer chemoprevention", J. Natl. Cancer Inst. 2003 Apr. 2; 95(7): 526-32, and by Vogel V.G. "The study of tamoxifen and raloxifene: preliminary enrolment data from a randomized breast cancer risk reduction trial", Clin Breast Cancer, 2002 Jun.; 3(2): 153-9.

The Framingham model is presented in the publications by Anderson K.M. et al., "Cardiovascular disease risk profiles", Am. Heart J. 1991 Jan; 121(1 Pt 2): 293-8 and by Wilson P.W.F. et al., "Prediction of coronary heart disease using risk factor categories", Circulation. 1998 May 12; 97(18): 1837-47. This Framingham model is commonly used by physicians for adapting a therapeutic prescription in cardiology and targeting prevention. The Bayer^{®} laboratories have, moreover, circulated a program that uses the Framingham algorithm; the physician enters onto the screen data relating to personal parameters of the individual to be treated, such as sex, age, weight, cholesterol level, etc., and the program provides a risk score representative of the possibilities of said individual being subject to a coronary disease, a cerebral stroke, etc.

The Gail and Framingham models are today used to define risk scores for populations different from the population represented by the initial prospective cohort. Now, it has been noted that, when such a model is crudely exported to a population different from the starting population, the resulting risk scores overestimate or underestimate the real risks. For example, for the French population, the Framingham algorithm, which was constructed from a prospective cohort of an American population, overestimates by a factor of 2 to 3 the risk of French people around 50 years old - Empana J.P. et al., "Étude de la validité des équations de risque cardiovasculaire, Étude PRIME", [Study of the validity of cardiovascular risk equations, PRIME study], Revue d'Epid et de Santé Pub, [Review of epidemiology and public health], October 2002.

To correct this bias, data from a cohort more representative of the population of the individual to be treated can be used. However, in the absence of such more representative cohorts, the physicians are often reduced to modifying one or other factor in order to reduce the bias - Laurier D. et al., "Estimation of CHD risk in a French working population using a modified Framingham model. The PCV-METRA Group." J. Clin. Epidemiol. 1994 Dec; 47(12): 1353-64. This bias correction can only be introduced when the factor modified has been identified as influencing the bias, but the correction is not often entirely satisfactory.

There exists therefore a need to reliably exploit the data available in existing cohorts on populations different from the initial prospective cohort.

There also exists a need for prospective cohorts that integrate more parameters and variables in order to be able to estimate risk scores with respect to various pathological conditions in addition to the risks of cardiovascular strokes and of breast cancer, and in particular for prospective cohorts that make it possible to measure the benefits and the risks associated with taking a given treatment that may result in side pathological conditions.

For example, the taking of hormone replacement therapies by a woman in pre-menopause, during menopause or after menopause is reputed to modify the risks of breast cancer, but also the risks of endometrial cancer, of ovarian cancer, of colon rectal cancer, of osteoporosis, of cardiovascular strokes and of Alzheimer's disease. There exists therefore a need to assess risk scores associated with these pathological conditions with and without hormone therapy in order to aid the physician in his or her prescription.

### SUMMARY OF THE INVENTION

To this effect, the invention proposes to use a prospective cohort and to estimate a relative risk for a given individual belonging to a population not represented by the cohort. This individual relative risk is then weighted by the incidence of the pathological condition in the population to which this individual belongs, so as to thus provide the absolute risk of this individual.

The crude application of equations defined for an initial population, and which result in the risks for an individual of another population being overestimated or underestimated, can thus be avoided.

The invention proposes more particularly a method of determining pathological condition risk scores for a given individual belonging to a final population, the method comprising the steps of:
- selecting a prospective cohort from an initial population;
- estimating an individual relative module of risk scores for the individual from the prospective cohort, said module comprising at least one risk score associated with at least one pathological condition variable;
- multiplying each risk score of the individual relative module by the incidence of each pathological condition in the final population of the individual.

According to one characteristic, the prospective cohort comprises:
- a selection of individuals belonging to an initial population;
- a catalogue of individual parameters for each individual; and
- a catalogue of pathological variables for each individual of the cohort having developed given pathological conditions.

According to one embodiment, the prospective cohort also comprises therapeutic parameters for each individual of the cohort that are associated with the taking of therapeutic treatments by said individual.

According to one embodiment, the therapeutic parameters comprise hormone replacement therapies.

According to one characteristic, the estimation of an individual relative module of risk scores comprises the steps of:
- searching for neighbours of the given individual in the prospective cohort, a neighbour being an individual of the cohort located at a distance from the given individual that is less than a threshold, a distance between individuals being determined from individual parameters;
- calculating means for each pathological variable over all the neighbours, weighted by each neighbour's distance to the given individual.

According to one characteristic, the risk score module comprises at least one risk score associated with a search for neighbours exhibiting zero therapeutic parameters and at least one risk score associated with a search for neighbours exhibiting non-zero therapeutic parameters.

According to one characteristic, the individual relative module of risk scores of the individual is multiplied by a relative risk due to a given treatment.

The invention also proposes a computer program that implements the method of the invention. The program comprises an interface for entering individual parameters associated with a given individual and selecting at least one pathological variable to be estimated, said program providing a module of individual relative risk scores associated with said pathological variable for said individual.

According to one embodiment, the interface makes it possible to enter the final population to which the given individual belongs, said program providing an absolute module of risk scores associated with said pathological variable for said individual.

According to one embodiment, the interface makes it possible to enter at least one therapeutic parameter associated with the taking of a therapeutic treatment, said program providing a matrix module of risk scores comprising at least one risk score associated with the pathological condition variable selected and at least one risk score associated with said pathological condition variable with taking of said therapeutic treatment.

### DETAILED DISCLOSURE OF THE INVENTION

The characteristics and advantages of the invention will become apparent on reading the following description of embodiments of the invention, given by way of example.

The invention proposes a method of determining pathological condition risk scores for a given individual belonging to a given final population. This method uses a prospective cohort constructed from an initial population, which may be different from the final population of the given individual.

The method proposes, first of all, carrying out an estimation of a risk score module for at least one given pathological condition, this risk score module being relative and individual for the given individual. This risk score module is termed relative since it is equal to the risk compared with the mean risk in the prospective cohort at the same age; it is estimated from the available prospective cohort, even if this prospective cohort is constructed from a population different from the population to which the given individual belongs. This risk score module is termed individual since it is estimated for a given individual based on the individual parameters thereof. The risk score module provides, for each individual, an estimation, often close to 1, of its relative risk (compared with the mean) that the given individual runs of developing a given pathological condition.

The method subsequently proposes multiplying each risk score of the individual relative module by the incidence of the pathological condition in the final population of the individual. Each module risk score estimation will therefore be weighted by a factor representative of the proportion of individuals affected, in one year, by the pathological condition in the final population of the given individual, thus resulting in an absolute risk.

In fact, the incidence of pathological conditions is generally very heterogeneous according to countries. The table below shows the incidence of breast cancer in France, in the United States and in Japan. The incidence represents the number of women having developed breast cancer per 100 000 women in the same age bracket. The data in the table for France can be consulted on the site http://www.esculape.com. The data_in the table for the United States are derived from the Surveillance Epidemiology and End Results, SEER, which can be consulted on the site http://www.seer.cancer.gov. The data in the table for Japan are taken from the report "Progress Report of the Research Group for Population-based Cancer Registration in Japan", 1996 and can be consulted on the site http://www.ncc.go.jp. In general, figures can also be found on http://www-dep.iarc.fr/, which depends on the WHO.

**Table I**

| Age | France | United States | Japan |
|---|---|---|---|
| 35-39 | 63 | 55 | 41 |
| 40-44 | 120 | 110 | 75 |
| 45-49 | 187 | 198 | 99 |
| 50-54 | 177 | 263 | 91 |
| 55-59 | 183 | 333 | 68 |
| 60-64 | 211 | 380 | 77 |
| 65-69 | 220 | 430 | 77 |
| 70-74 | 232 | 476 | 71 |
| 75-79 | 220 | 499 | 72 |
| All ages | 89 | 137 | 43 |

It is readily noted from Table 1 that, for a Japanese woman, an individual absolute risk score calculated from an American population cohort would provide an estimation of breast cancer risks that is much too high compared with the risks run by this Japanese woman, even though many individual parameters are similar between this Japanese woman and certain women of the American cohort having developed breast cancer.

In the absence of a prospective cohort constructed from the Japanese population, the method of the invention proposes selecting a prospective cohort constructed on another population, determining an individual relative module of risk scores and multiplying the scores of this module by the incidences of the pathological conditions under consideration.

The method proposed therefore comprises a first step of selection of a prospective cohort. The method of the invention can in fact exploit an already constructed prospective cohort, for example one of the cohorts constructed from the American population. The method of the invention can also exploit a new cohort constructed, for example, from 100000 individuals derived from the French population and followed for 15 years. Such a cohort is known under the name E3N and the articles published in its respect can be consulted on the site http://www.e3n.net/.

This prospective cohort is said to be based on an initial population as opposed to the final population in which the individual whose risks it is desired to calculate will be located. The individuals selected for constituting the prospective cohort are then followed over many years and the pathological conditions that they develop are catalogued.

The prospective cohort integrates individual parameters specific to each individual of the initial population integrated into the cohort. These individual parameters for each individual of the prospective cohort may comprise age, weight, sex, eating behaviour parameters, smoking, number of children, age when periods first started, cholesterol level and other relevant parameters. Some of these parameters will remain constant for a given individual and others will be variable, i.e. information relating thereto will be updated as a function of the ageing of the individual monitored in the cohort.

The cohort also includes pathological variables which represent the pathological conditions developed by the individuals of the cohort. The number of individuals who have developed a given pathological condition is therefore correlated with individual parameters.

It is also proposed to include, in addition to the individual parameters mentioned above, therapeutic parameters relating to treatments followed by the individuals of the cohort, in order to be able to correlate the pathological conditions developed with the taking of a given treatment. In particular, it is proposed to consider, as a therapeutic parameter, the taking of a given hormone replacement therapy. The E3N cohort mentioned above includes such parameters. Such a prospective cohort then subsequently allows physicians to assess the risks relating to the taking of any hormone therapy compared with the risks without treatment for the same pathological condition. In fact, in the case of hormone replacement therapies (HRT), it is necessary to individually assess the benefit/risk by quantifying the impact of the taking of HRT on each pathological condition risk.

A new program can then be created using this new prospective cohort which integrates a database including therapeutic parameters associated with the taking of any treatment in addition to the "conventional" individual parameters of age, behaviour and history. The database also includes the pathological variables catalogued in the prospective cohort. The program could thus provide risk scores correlated with the various hormone therapies identified in the cohort.

A physician faced with a patient in pre-menopause could thus provide, as program input data, the personal variables of the patient corresponding to the personal parameters of the cohort. The program would then provide risk scores for various pathological conditions in the case where the patient would be taking no hormone replacement therapy (HRT) and in the case where she would be taking any such therapy. A program output could have the form of the table II below - the figures indicated are examples of presentation of results and do not come from a real simulation case.

**Table II**

| Risk at 10 years | Without HRT | Taking of oestrogen | Taking of micro nized progesterone | Taking of synthetic progesterone |
|---|---|---|---|---|
| Breast cancer | 3,5% | 4% | 3% | 5% |
| Ovarian cancer | 0,5 % | | | |
| Colon cancer | 0,5 % | | | |
| Osteoporosis | 2 % | | | |
| Cardiovascular | 7 % | | | |
| Alzheimer's | 6 % | | | |

The physician could thus measure the benefit and the risk of taking any such therapy as a function of the personal parameters of the patient, and choose whether or not HRT should be taken, and if yes, which one.

The risk scores provided by such a program would be calculated by the method of the invention, i.e. by estimating first of all an individual relative module of risk scores for each of the pathological conditions listed in the first column of Table II, and then multiplying each risk score of the module by the incidence of each pathological condition in the population to which the patient belongs, if said patient belongs to a population different from that of the prospective cohort used.

Based on the prospective cohort selected, the method proposes considering whether the given individual belongs to the same population as this prospective cohort. If such is the case, an estimation of the risks associated with any such pathological condition may be performed by searching for neighbours in the prospective cohort or by a parametric method, as for the scores derived from the Framingham cohort and described in the publication by Wilson P.W.F., et al. "Prediction of coronary heart disease using risk factor categories". Circulation 1998;97: 1837-1847. On the other hand, if the given individual does not belong to the same population as the prospective cohort selected, a score estimated in the prospective cohort may result in the risks associated with this pathological condition being underestimated or overestimated, as explained with reference to Table I. An estimation of a relative risk before multiplication by the incidence of the pathological condition in the population to which the individual to be treated belongs will then be necessary.

The method therefore proposes estimating first of all a relative and individual module of risk scores, for example by performing a search for neighbours on the prospective cohort.

The expression "neighbouring region of a given individual" is intended to mean all the individuals of the cohort that exhibit substantially the same personal parameters. A distance D between the given individual I and an individual C of the cohort is then defined, for example by calculating the sum of the squares of the differences between each individual parameter of the individual I and of a member of the cohort C. A screen may be applied beforehand in order to exclude certain individual or therapeutic parameters that are known to be insignificant for the pathological variable to be determined. For example, if the intention is to estimate the risks of breast cancer, the personal parameters for cholesterol may be excluded. Different weights can also be attributed to the various individual and therapeutic parameters selected. Once the distances D have been calculated between the individual I and the members of the cohort C, a limiting threshold Dₗᵢₘ is fixed, below which a member of the cohort C will be considered to be a neighbour V. The importance of the neighbours can subsequently be weighted as a function of their distance to the given individual I.

The estimation of the individual absolute risk score associated with a given pathological condition for a given individual I may then be a weighted mean of the pathological condition variable over all the neighbours V of the cohort. Such a risk score estimation may be carried out for each pathological condition to be examined for the given individual I. The risk scores for various pathological conditions can be estimated from the same neighbouring region, or the neighbouring region can be calculated for each pathological condition variable to be clarified by applying different screens for excluding certain individual or therapeutic parameters. A risk score module representative of the risks associated with various pathological conditions that may be developed by the individual I who is still healthy is thus obtained; this module of absolute risk scores becomes relative by dividing by the mean risks at the same age in the cohort and individual with respect to the parameters of the given individual I.

The individual relative module of risk scores could also be determined by a parametric method as mentioned above with reference to the Framingham cohort.

As indicated above, if the given individual I belongs to the same population as its neighbours V of the cohort, this module of absolute risk scores constitutes a good estimation of the risks that this individual runs of developing any pathological condition. However, if said individual I belongs to another population, the risk scores determined as indicated above are biased, as the previously discussed Table I shows. The risk score module to be used must be a relative module by dividing by the mean incidences in the cohort. The method therefore proposes multiplying each score of the individual relative risk module by the incidence of the pathological condition in the population to which the given individual I belongs. The incidence of each pathological condition can be determined as the ratio of the number of individuals having annually developed said pathological condition in the final population to the total number of individuals in said population for the same age bracket.

An annual absolute risk is thus obtained. For a risk at 10 years of a 50-year old woman, the relative risk will be multiplied by the sum of the incidences at 50 years, at 51 years, etc. up to 60 years, if precise yearly incidences per age are available. In general, incidence values are available by age ranges. The relative risk will then be multiplied by the sum of the incidences by age brackets.

The method of the invention may be implemented by computer. A program distributed to physicians may have an interface by means of which the physician enters a certain number of individual parameters specific to his or her patient and selects the pathological variables intended to be determined. The program will provide a risk score module comprising a series of risk scores associated with a series of pathological conditions selected by the physician.

The physician can select a population option if the patient belongs to a population other than the population from which the prospective cohort used to program the program is derived; the physician can thus tick the population to which his or her patient belongs among a choice of proposed populations. The program will then weight the individual relative module of risk scores with respect to the incidences of each pathological condition selected by the physician, so as to provide an absolute module of risk scores.

Another option can also be selected if the physician envisages prescribing his or her patient any given therapeutic treatment; the physician can tick one or more boxes corresponding to the therapeutic treatments envisaged. The program will then calculate an individual relative module of risk scores in the form of a matrix comprising a series of risk scores associated with the pathological conditions selected by the physician and series of risk scores associated with said pathological conditions with the taking of any therapeutic treatment, as shown in Table II.

According to one embodiment, the program can be programmed so as to calculate the risk for a woman receiving a specific treatment, for example receiving estrogens, by considering that it is one variable like another. According to another embodiment, the program calculates a risk without treatment, which will subsequently be multiplied by the relative risk due to the estrogens, for example 1.4 if the relative risk data presented in the article by Fournier et al., "Breast cancer risk in relation to different types of hormone replacement therapy in the E3N-EPIC cohort", Int. J. Cancer. 2005 Apr 10; 114(3):448-54, are used. The publication selected for determining the relative risk of a treatment must be chosen such that its study population is close to the final population. The expression "relative risk of a treatment" is intended to mean the risk of a population receiving treatment compared with a population not receiving treatment; this relative treatment risk should not be confused with the individual relative risk defined above.

The method of the invention therefore allows a more reliable estimation of the pathological condition risk scores for individuals belonging to populations different from the initial population of the prospective cohort, and also makes it possible to estimate the impact of therapeutic treatments on these risk scores.

## Claims

1. Method of determining pathological condition risk scores for a given individual belonging to a final population, the method comprising the steps of:
- selecting a prospective cohort from an initial population;
- estimating an individual relative module of risk scores for the individual from the prospective cohort, said module comprising at least one risk score associated with at least one pathological condition variable;
- multiplying each risk score of the individual relative module by the incidence of each pathological condition in the final population of the individual.

2. Method of Claim 1, in which the prospective cohort comprises:
- a selection of individuals belonging to an initial population;
- a catalogue of individual parameters for each individual; and
- a catalogue of pathological variables for each individual of the cohort having developed given pathological conditions.

3. Method of Claim 2, in which the prospective cohort also comprises therapeutic parameters for each individual of the cohort that are associated with the taking of therapeutic treatments by said individual.

4. Method of Claim 3, in which the therapeutic parameters comprise hormone replacement therapy.

5. Method of one of Claims I to 4, in which the estimation of an individual relative module of risk scores comprises the steps of:
- searching for neighbours of the given individual in the prospective cohort, a neighbour being an individual of the cohort located at a distance from the given individual that is less than a threshold, a distance between individuals being determined from individual parameters;
- calculating means for each pathological variable over all the neighbours, weighted by each neighbour's distance to the given individual.

6. Method of Claims 5 and 3, in which the risk score module comprises at least one risk score associated with a search for neighbours exhibiting zero therapeutic parameters and at least one risk score associated with a search for neighbours exhibiting non-zero therapeutic parameters.

7. Method of one of Claims 1 to 6, in which the individual relative module of risk scores of the individual is multiplied by a relative risk due to a given treatment.

8. Computer program that implements the method of one of Claims 1 to 7, comprising an interface for entering individual parameters associated with a given individual and selecting at least one pathological variable to be estimated, said program providing a module of individual relative risk scores associated with said pathological variable for said individual.

9. Program of Claim 8, in which the interface makes it possible to enter the final population to which the given individual belongs, said program providing an absolute module of risk scores associated with said pathological variable for said individual.

10. Program of Claim 8 or 9, in which the interface makes it possible to enter at least one therapeutic parameter associated with the taking of a therapeutic treatment, said program providing a matrix module of risk scores comprising at least one risk score associated with the pathological condition variable selected and at least one risk score associated with said pathological condition variable with taking of said therapeutic treatment.
